# EUROPÄISCHE PATENTANMELDUNG

(11) **EP 1 371 625 A1**
(43) Veröffentlichungstag der Anmeldung: **17.12.2003**
(21) Anmeldenummer: 03012421.8
(22) Anmeldetag: 30.05.2003
(51) Int. Cl.: C07C 51/00, C07C 67/08, C07C 65/03, C07C 69/86

(54) **Verfahren zur Herstellung von 3-Acyloxy-2-methylbenzoesäuren**

(30) Priorität: 13.06.2002 DE 10226219
(71) Anmelder: Bayer Aktiengesellschaft, 51368 Leverkusen (DE)
(72) Erfinder: Scherer, Johannes, Dr., 51375 Leverkusen (DE); Behre, Horst, Dr., 51519 Odenthal (DE); Müller-Hauck, Friedrich, Dr., 25557 Bendorf (DE)

(57) **Zusammenfassung**

Die Erfindung betrifft ein verbessertes Verfahren zur Herstellung von 3-Acyloxy-2-methylbenzoesäuren durch Erhitzen von substituierten Naphthalinen in Gegenwart von Alkalimetallhydroxiden und anschließender Acylierung.

## Beschreibung

Die Erfindung betrifft ein verbessertes Verfahren zur Herstellung von 3-Acyloxy-2-methylbenzoesäuren durch Erhitzen von substituierten Naphthalinen in Gegenwart von Alkalimetallhydroxiden und anschließender Acylierung.

3-Acyloxy-2-methylbenzoesäuren, wie zum Beispiel 3-Acetoxy-2-methylbenzoesäure sind wertvolle Zwischenprodukte bei der Herstellung von Arzneimitteln, wie zum Beispiel HIV-Protease-Inhibitoren (siehe z.B. US-A 5,484,926) und Agrochemikalien.

Aus EP-A 891 964 ist ein Verfahren bekannt, das ausgehend von 3-Amino-1,5-naphthalindisulfonsäure oder deren Alkalimetallsalzen über die Umsetzung mit Alkalimetallhydroxid, Abtrennung von Unlöslichem und Reaktion mit Acetanhydrid verläuft.

Nachteilig an diesem Verfahren ist der Einsatz der teuren 3-Amino-1,5-naphthalindisulfonsäure oder deren Salze sowie die nicht zufriedenstellende Gesamtausbeute von 60 %.

Es bestand daher das Bedürfnis, ein effizientes Verfahren zu entwickeln, das die Herstellung von 3-Acyloxy-2-methylbenzoesäuren in vorteilhafter Weise ermöglicht.

Es wurde nun ein Verfahren zur Herstellung von Verbindungen der Formel (I) gefunden, 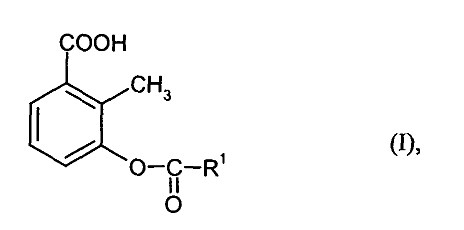 in der
R¹ für C₁-C₂₀-Alkyl, C₇-C₂₀-Arylalkyl, C₁₃-C₂₀-Diarylalkyl oder Reste der Formeln (IIa) oder (IIb) steht

A-OR² (IIa)

A-NR³R⁴ (IIb)

in der A jeweils für einen C₁-C₄-Alkylenrest und R² sowie R³ und R⁴ unabhängig voneinander jeweils für Methyl, Ethyl und Isopropyl stehen,
das dadurch gekennzeichnet ist, dass
a) Verbindungen der Formel (III) 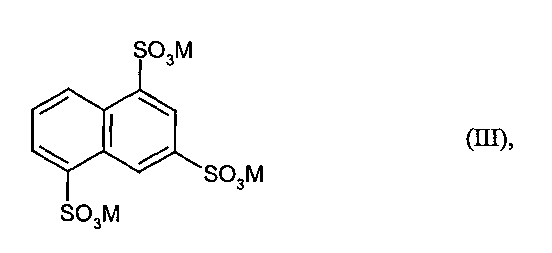 in der M jeweils unabhängig für Wasserstoff, Ammonium, ein Alkalimetall oder ein halbes Äquivalent eines Erdalkalimetalls stehen kann,
   mit Alkalimetallhydroxid und gegebenenfalls Erdalkalimetallhydroxid in Gegenwart von Wasser umgesetzt werden und
b) die in Schritt a) erhaltenen Reaktionsmischungen,
   - gegebenenfalls nach Zugabe von Wasser und
   - gegebenenfalls nach der Abtrennung von unlöslichen Bestandteilen und
   - gegebenenfalls nach der Abtrennung von unerwünschten, löslichen Bestandteilen
      teilweise neutralisiert werden und
c) die in Schritt b) erhaltenen Reaktionsmischungen mit Verbindungen der Formeln (IVa), oder (IVb)

   R¹-COX (IVa)

   (R¹-CO)₂O (IVb)

   in denen die Reste R¹ jeweils unabhängig voneinander die oben genannte Bedeutung besitzen
   umgesetzt werden und
d) die in Schritt c) erhaltenen Reaktionsmischungen angesäuert werden.

Alkyl bzw. Alkylen bedeutet im Rahmen der Erfindung jeweils unabhängig einen geradkettigen, cyclischen, verzweigten oder unverzweigten Alkyl- bzw. Alkylen-Rest. Gleiches gilt für den Alkylenteil eines Arylalkyl-Restes.

Beispielsweise steht in allen Zusammenhängen bevorzugt C₁-C₂₀-Alkyl für Methyl, Ethyl, n-Propyl, Isopropyl, n-Butyl, tert.-Butyl, n-Pentyl, Cyclohexyl, n-Hexyl, n-Heptyl, n-Octyl, iso-Octyl, n-Decyl, n-Dodecyl, n-Hexadecyl und n-Octadecyl.

Beispielsweise steht C₁-C₄-Alkylen in allen Zusammenhängen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen.

**Arylalkyl** steht im Rahmen der Erfindung beispielsweise und bevorzugt für Alkylreste, die durch carbocyclische aromatische Reste mit 6 bis 10 Kohlenstoffatomen wie insbesondere Phenyl und Naphthyl substituiert sind, wobei die carbocyclischen aromatischen Reste ihrerseits mit bis zu fünf Substituenten pro Cyclus substituiert sein können, die ausgewählt sind aus der Gruppe Methyl, Ethyl, Fluor, Chlor, Brom und C₁-C₄-Fluoralkyl, wobei Fluoralkyl für einen einfach mehrfach oder vollständig durch Fluor substituierten Alkylrest gemäß obiger Definition steht. Gleiches gilt für den Arylteil eines Diarylalkyl-Restes.

Insbesondere steht C₁-C₄-Fluoralkyl für Trifluormethyl, Pentafluorethyl, 2,2,2-Trifluorethyl und Nonafluorbutyl.

Besonders bevorzugt werden für Schritt a) 1,3,5-Naphtalintrisulfonsäure und deren Mono-, Di- oder Tri-Alkalimetallsalze eingesetzt.

Ganz besonders bevorzugt werden die Trialkalimetallsalze der 1,3,5-Naphtalintrisulfonsäure wie beispielsweise Trinatrium-1,3,5-naphtalin-trisulfonat und Trikalium-1,3,5-naphtalin-trisulfonat eingesetzt, wobei Trinatrium-1,3,5-naphtalin-trisulfonat noch weiter bevorzugt ist.

Einige der Verbindungen der Formel (III) können in Form von Hydraten auftreten, die nichts eigens erwähnt werden, aber im Rahmen der Erfindung mitumfasst sind.

Die Verbindungen der Formel (III) sind entweder kommerziell verfügbar, oder nach Literaturvorschriften oder analog dazu herstellbar.

In einer bevorzugten Ausführungsform des erfindungsgemäßen Verfahrens werden die Naphthalin-1,3,5-trisulfonsäure oder deren Mono-, Di- oder Tri-Alkalimetallsalze so hergestellt dass
i) Naphthalin mit rauchender Schwefelsäure zu Naphthalin-1,3,5-trisulfonsäure umgesetzt wird und
ii) die in Schritt i) erhaltene Naphthalin-1,3,5-trisulfonsäure gegebenenfalls mit einer alkalimetallhaltigen Base in ein Mono-, Di- oder Tri-Alkalimetallsalz überführt wird.

An dieser Stelle sei darauf hingewiesen, dass beliebige Kombination der Merkmale und der genannten Vorzugsbereiche ebenfalls vom Rahmen der Erfindung mitumfasst sind.

Schritt i) kann vorteilhafterweise durch Umsetzung von Naphthalin mit rauchender Schwefelsäure erfolgen.

Beispielsweise kann man dabei so vorgehen, dass man rauchende Schwefelsäure vorlegt und Naphthalin zugibt oder konzentrierte Schwefelsäure und Naphthalin vorlegt und rauchende Schwefelsäure zugibt oder konzentrierte Schwefelsäure vorlegt und Naphthalin und rauchende Schwefelsäure zugibt.

Bevorzugt geht man im Schritt i) so vor, dass man konzentrierte Schwefelsäure vorlegt und Naphthalin und rauchende Schwefelsäure zugibt.

Konzentrierte Schwefelsäure bedeutet im Rahmen der Erfindung beispielsweise eine Schwefelsäure mit 90 bis 100 Gew.-% H₂SO₄. Rauchende Schwefelsäure bedeutet im Rahmen der Erfindung eine Schwefelsäure, die einen Gehalt von über 100 Gew.-% bezogen auf reine H₂SO₄ besitzt. Eine gängige Bezeichnung für rauchende Schwefelsäure im Sinne der Erfindung ist auch Oleum.

Üblicherweise wird bei handelsüblichem Oleum der Gehalt an freiem SO₃ angegeben, der beispielsweise 30 oder 65 Gew.-% beträgt.

Bevorzugt verwendet man im Schritt a) soviel Oleum, dass das molare Verhältnis von freiem SO₃ zu Naphthalin zwischen 1,5:1 und 10:1, bevorzugt zwischen 2:1 und 5:1 und besonders bevorzugt zwischen 2,5:1 und 4:1 liegt.

Die Temperatur bei der Zugabe kann beispielsweise -20 bis 70°C betragen, 20 bis 55°C sind bevorzugt.

Die Zeit für die Zugabe kann beispielsweise zwischen 10 min und 48 h liegen, bevorzugt sind 2h bis 24 Stunden.

Anschließend kann die resultierende Reaktionsmischung gegebenenfalls noch erhitzt werden. Die Temperatur kann dabei beispielsweise zwischen 55 und 150°C bevorzugt zwischen 80 und 100°C liegen.

Aus der resultierenden Reaktionsmischung kann die Naphthalin-1,3,5-trisulfonsäure zum Beispiel durch Zugabe von Wasser gewonnen werden.

Die Herstellung von Alkali- oder Erdalkalimetallsalzen der Naphthalin-1,3,5-trisulfonsäure gemäß Schritt ii) kann entweder aus der isolierten Naphthalin-1,3,5-trisulfonsäure oder direkt aus der resultierenden Reaktionsmischung aus Schritt i) erfolgen. Die Herstellung von Alkali- oder Erdalkalimetallsalzen der Naphthalin-1,3,5-trisulfonsäure aus der resultierenden Reaktionsmischung aus Schritt i) ist bevorzugt.

Schritt ii) kann beispielsweise derart erfolgen, dass die resultierende Reaktionsmischung aus Schritt i) z.B. durch Gießen in Wasser oder auf Eis verdünnt wird, und anschließend mit Alkalimetallhydroxiden, -hydrogencarbonaten oder -carbonaten oder wässrige Lösungen davon umgesetzt wird.

Bevorzugt werden Alkalimetallhydroxide wie insbesondere Natriumhydroxid und Kaliumhydroxid oder deren wässrige Lösungen eingesetzt.

Besonders bevorzugt ist die Umsetzung mit wässrigen Lösungen von Natriumhydroxid.

Der Gehalt der Lösungen an Alkalimetallhydroxiden kann beispielsweise zwischen 2 und 75 Gew.-% betragen, bevorzugt sind 25 bis 60 Gew.-%.

Die Temperatur der Umsetzung für Schritt ii) kann beispielsweise 0 bis 100°C betragen, bevorzugt sind 80 bis 100°C.

Die Menge des eingesetzten Alkalimetallhydroxids kann beispielsweise das 2 bis 10 fache bezogen auf das molare Verhältnis des in Schritt i) eingesetzten Naphthalins sein, bevorzugt das 2,8 bis 3,5 fache.

Nach in an sich bekannter Aufarbeitung, die zum Beispiel durch Filtration und gegebenenfalls Waschen und Trocknung des ausgefallenen Feststoffs erfolgen kann erhält man Alkalimetallsalze der Naphthalin-1,3,5-trisulfonsäure, die entweder gelagert oder vorzugsweise weiter umgesetzt werden kann.

Gegebenenfalls kann das gemäß Schritt ii) erhaltene Alkalimetallsalz der Naphthalin-1,3,5-trisulfonsäure z.B. durch Umkristallisation noch weiter gereinigt werden, für den Einsatz in Schritt a) des erfindungsgemäßen Verfahrens ist dies jedoch nicht nötig.

Im **Schritt a)** des erfindungsgemäßen Verfahrens werden die Verbindungen der Formel (III) mit Alkalimetallhydroxid und gegebenenfalls Erdalkalimetallhydroxid in Gegenwart von Wasser umgesetzt.

Als Alkalimetallhydroxid können beispielsweise und bevorzugt Natrium- und Kaliumhydroxid oder Mischungen davon z.B. als Feststoff und in Form wässriger Lösungen eingesetzt werden.

Die Menge an Alkalimetallhydroxid für Schritt a) kann beispielsweise so gewählt werden, dass pro mol der Verbindung der Formel (III) 6 bis 40 mol, bevorzugt 8 bis 25 mol Alkalimetallhydroxid eingesetzt werden.

Bevorzugt kann weiterhin auch der Zusatz von Erdalkalimetallhydroxiden erfolgen. Geeignete Erdalkalimetallhydroxide sind beispielsweise Magnesiumhydroxid und Calciumhydroxid, bevorzugt ist Calciumhydroxid.

Die Menge an Erdalkalimetallhydroxid für Schritt a) kann beispielsweise so gewählt werden, dass pro mol der Verbindung der Formel (III) 0,5 bis 30 mol, bevorzugt 2 bis 20 mol, besonders bevorzugt 2 bis 15 mol und ganz besonders bevorzugt 2,0 bis 3,1 mol Erdalkalimetallhydroxid eingesetzt werden.

Das molare Verhältnis von Wasser zur Verbindung der Formel (III) kann beispielsweise 0,5 bis 200 mol, bevorzugt 3 bis 50 mol betragen.

In einer besonders bevorzugten Ausführungsform beträgt das Verhältnis von Wasser zu der Summe aus Alkalimetallhydroxid und gegebenenfalls zugesetztem Erdalkalimetallhydroxid 1:1,4 bis 1:6,0.

Der Druck bei der Reaktion kann beispielsweise 1 bis 200 bar betragen, bevorzugt 1 bis 100 bar, besonders bevorzugt 10 bis 60 bar und ganz besonders bevorzugt derjenige Druck, der sich durch Aufheizen des Reaktionsgemisches auf die Reaktionstemperatur in einem geschlossenen Gefäß ausgehend von Umgebungsdruck einstellt.

Als geschlossenes Gefäß kommt zum Beispiel ein Autoklav in Frage, der z.B. aus Nickel, Nickel-Basislegierungen, Silber oder anderen, gegen Alkalien resistenten Material sein kann.

Die Temperatur der Umsetzung kann beispielsweise 240 bis 350°C betragen, bevorzugt 270 bis 320°C.

Die Reaktionszeit kann beispielsweise 2 bis 25 Stunden, bevorzugt 3 bis 8 Stunden betragen.

Im **Schritt b)** des erfindungsgemäßen Verfahrens werden die gemäß Schritt a) erhaltenen Reaktionsmischungen gegebenenfalls nach Zugabe von Wasser und gegebenenfalls nach der Abtrennung von unlöslichen Bestandteilen und gegebenenfalls der Abtrennung von unerwünschten, löslichen Bestandteilen mit Säure zumindest teilweise neutralisiert.

In einer bevorzugten Ausführungsform wird gegebenenfalls nach Abkühlen der Reaktionsmischung Wasser zugesetzt, was z.B. durch Eingießen der Reaktionsmischung in Wasser oder auf Eis erfolgen kann.

Weiterhin bevorzugt werden unlösliche Bestandteile abgetrennt. Das kann beispielsweise und bevorzugt durch Filtration, Zentrifugation, Sedimentation und Dekantieren gegebenenfalls in Gegenwart von Hilfsmitteln erfolgen. Hilfsmittel können beispielsweise sein Kieselgur, wie zum Beispiel Celite ®, Aktivkohle, wie zum Beispiel Norite ®, Bleicherde, Montmorillonit oder Knochenkohle erfolgen. Bevorzugt ist die Filtration, besonders bevorzugt die Filtration in Gegenwart von Hilfsmitteln, bevorzugt Aktivkohle.

Ebenfalls bevorzugt ist die Abtrennung unerwünschter, löslicher Bestandteile. Unerwünschte, lösliche Bestandteile sind ohne Anspruch auf Vollständigkeit z.B. gefärbte, organische Nebenprodukte. Die Abtrennung unerwünschter, löslicher Bestandteile kann beispielsweise vor oder nach der zumindest teilweisen Neutralisation vorgenommen werden. Dazu geht man beispielsweise so vor, dass mit organischen Solvens extrahiert wird. Geeignete organische Solventien sind beispielsweise Ester wie Essigester und Butylacetat, aliphatische oder aromatische, gegebenenfalls halogenierte Kohlenwasserstoffe wie beispielsweise Benzin, Benzol, Toluol, Xylole, Chlorbenzol, Dichlorbenzole, Iso-propylbenzol, Petrolether, Hexan, Heptan, Octan, iso-Octan, Cyclohexan, Methylcyclohexan, Dichlormethan, Chloroform oder Tetrachlorkohlenstoff; Ether, wie Diethylether, Methyl. tert. Butylether oder Diisopropylether, Ketone, wie 2-Butanon oder Methyl-isobutyl-keton oder Mischungen solcher Lösungsmittel.

Weiterhin kann die Reaktionsmischung mit einem geeigneten Adsorbens z.B. von Verfärbungen befreit werden. Geeignete Adsorbentien sind beispielsweise Kieselgele, Aluminiumoxide, Cellulose oder Aktivkohle.

Der pH-Wert kann bei der Entfernung unerwünschter, löslicher Bestandteile beispielsweise 4 bis 13, bevorzugt 6,5 bis 11,5 betragen. Bevor Schritt c) durchgeführt wird kann im Falle von pH-Werten unter 7,5 wieder Base zugegeben werden um den bevorzugten pH-Bereich in Schritt a) zu erreichen.

Die teilweise Neutralisation erfolgt bevorzugt dadurch, dass auf einen pH-Wert von 7,5 bis 13, bevorzugt 9,5 bis 11,5 eingestellt wird. Die pH-Wert-Angaben beziehen sich dabei auf Werte bei 25°C. Vorzugsweise werden zur teilweisen Neutralisation Säuren oder saure Salze mit einen mit einem pKa-Wert in Wasser von 5 oder weniger eingesetzt. Bevorzugt werden zur teilweisen Neutralisation Schwefelsäure, Salzsäure, Phosphorsäure, Salpetersäure und Bromwasserstoffsäure besonders bevorzugt Schwefelsäure und Salzsäure, ganz besonders bevorzugt Schwefelsäure eingesetzt.

In einer ganz besonders bevorzugten Ausführungsform wird Schritt b) so durchgeführt, dass das aus Schritt a) erhaltene Reaktionsgemisch zunächst mit Wasser verdünnt wird, unlösliche Bestandteile abgetrennt werden, unerwünschte, lösliche Bestandteile abgetrennt werden und ein pH-Wert von 7,5 bis 13 eingestellt wird.

Im **Schritt c)** erfolgt die Umsetzung des Reaktionsgemisches aus Schritt b) mit Verbindungen der Formeln (IVa) oder (IVb).

In den Verbindungen der Formeln (IVa) steht R¹ bevorzugt für Methyl, Ethyl, n-Propyl, iso-Propyl, tert.-Butyl oder Benzyl, besonders bevorzugt für Methyl und X für Chlor, Brom oder Iod.

Besonders bevorzugte Verbindung der Formel (IVa) ist Acetylchlorid.

In den Verbindungen der Formeln (IVb) steht R¹ jeweils unabhängig, bevorzugt identisch für Methyl, Ethyl, n-Propyl, iso-Propyl oder Benzyl, besonders bevorzugt für Methyl.

Besonders bevorzugte Verbindung der Formel (IVb) und für das erfindungsgemäße Verfahren besonders bevorzugt ist Acetanhydrid.

Die Menge an Verbindung der Formel (IVa) oder (IVb) wird beispielsweise und bevorzugt so gewählt, dass das molare Verhältnis zur ursprünglich eingesetzten Verbindung der Formel (II) 0,8:1 bis 10:1, bevorzugt 1:1 bis 5:1 beträgt.

Die Reaktionstemperatur in Schritt c) beträgt beispielsweise -10 bis 120°C, bevorzugt -10-100°C und besonders bevorzugt 50 bis 100°C.

Der Reaktionsdruck für Schritt c) ist nicht kritisch, bevorzugt ist Umgebungsdruck.

Die Reaktionszeit für Schritt c) kann beispielsweise 1 bis 25 Stunden betragen, bevorzugt sind 2 bis 10 Stunden.

Weiterhin ist bevorzugt, den pH-Wert im Laufe der Reaktion zwischen 7,5 und 13, bevorzugt 9,5 und 11,5 zu halten, was beispielsweise durch Zugabe von Base erfolgen kann.

Als Base eignen sich insbesondere Alkalimetallhydroxide, -carbonate oder -hydrogencarbonate, wobei Natriumhydroxid und Kaliumhydroxid bevorzugt sind. Der Einsatz der Base kann beispielsweise in fester Form oder in Form wässriger Lösungen erfolgen.

Anschließend wird im **Schritt d)** die im Schritt c) erhaltene Reaktionsmischung angesäuert.

Bevorzugt wird auf einen pH-Wert von 3,5 oder weniger, besonders bevorzugt 0 bis 3,5 und ganz besonders bevorzugt 1 bis 2,5 angesäuert.

Zum Ansäuern eignen sich beispielsweise Säuren oder saure Salze mit einem pKa-Wert in Wasser von 3,5 oder weniger, bevorzugt 0 oder weniger. Besonders bevorzugt werden Schwefelsäure oder Salzsäure eingesetzt.

In einer bevorzugten Ausführungsform im Schritt d) wird zunächst nur auf einen pH-Wert von über 3,5 bis unter 8 angesäuert und unerwünschte, lösliche Bestandteile analog wie oben beschrieben extraktiv entfernt. Anschließend kann die Reaktionsmischung dann mit einem geeigneten Adsorbens z.B. von Verfärbungen befreit werden. Geeignete Adsorbentien sind beispielsweise Kieselgele, Aluminiumoxide, Cellulose oder Aktivkohle.

Anschließend wird dann weiter angesäuert.

Die Temperatur beim Ansäuern ist nicht kritisch, es kann jedoch vorteilhaft sein, die Reaktionsmischung zum Sieden zu erhitzen um gelöste Gase auszutreiben oder gegebenenfalls vorhandenes Sulfit zu zersetzen. Durch das Ansäuern werden die Monooder (disalze) Salze der Verbindungen der Formel (I) protoniert und zumindest teilweise in die freien Säuren der Formel (I) überführt.

Aus den gemäß Schritt d) erhaltenen Reaktionsgemischen können die Verbindungen der Formel (I) in an sich bekannter Weise beispielsweise durch Extraktion mit organischem Lösungsmittel, Filtration, Zentrifügation oder Sedimentation und Dekantieren gewonnen werden.

Für die Extraktion bevorzugte Lösungsmittel sind beispielsweise Ester wie Essigester und Butylacetat, aliphatische oder aromatische, gegebenenfalls halogenierte Kohlenwasserstoffe wie beispielsweise Benzin, Benzol, Toluol, Xylole, Chlorbenzol, Dichlorbenzole, Iso-propylbenzol, Petrolether, Hexan, Heptan, Octan, iso-Octan, Cyclohexan, Methylcyclohexan, Dichlormethan, Chloroform oder Tetrachlorkohlenstoff; Ether, wie Diethylether, Methyl. tert. Butylether oder Diisopropylether, Ketone, wie 2-Butanon oder Methyl-isobutyl-keton oder Mischungen solcher Lösungsmittel.

Die Verbindungen der Formel (I) können nach Extraktion in an sich bekannter Weise beispielsweise durch Verdampfen des Lösungsmittels gewonnen werden.

Trennt man die Verbindungen der Formel (I) durch Filtration, Zentrifugation oder Sedimentation und Dekantieren ab, so kann dies beispielsweise und bevorzugt bei -20 bis 70°C, besonders bevorzugt 5 bis 55°C geschehen.

Zur weiteren Reinigung können die Verbindungen der Formel (I) gegebenenfalls umkristallisiert oder umgefällt werden, nötig ist dies jedoch nicht.

Die nach den erfindungsgemäßen Verfahren hergestellten Verbindungen der Formel (I) wie insbesondere 3-Acetoxy-2-methylbenzoesäure eignen sich besonders zur Verwendung in einem Verfahren zur Herstellung von Arzneimitteln wie zum Beispiel HIV-Proteaseinhibitoren und Agrochemikalien wie zum Beispiel Pflanzenschutzmitteln und Insektiziden oder Zwischenprodukten davon wie insbesondere den Säurechloriden oder Säurebromiden.

Weiterhin eignen sich die Verbindungen der Formel (I) zur Herstellung der analogen aktivierten Säurederivate wie insbesondere den Säurechloriden und Säurebromiden.

Der Vorteil der erfindungsgemäßen Verfahren ist die effiziente Herstellung von Verbindungen der Formel (I), die die Durchführung ohne aufwändige Zwischenisolierungen in hohen Ausbeuten ermöglicht.

### Beispiele

### Beispiel 1

In einem Nickelautoklaven werden in ein Gemisch aus 232,0 g wässriger Natronlauge (45 %ig) und 194,0 g Natriumhydroxid 300,0 g Trinatrium-1,3,5-naphthalintrisulfonat (70,9 %ig, berechnet als freie Säure) so eingerührt, dass eine gut rührbare, breiartige Suspension entsteht. Der Autoklav wird verschlossen und auf 280°C aufgeheizt. Der Innendruck (Eigendruck) steigt dabei auf 18,4 bar. Es wird 6 Stunden bei Reaktionstemperatur nachgerührt und danach auf 90°C abgekühlt. Bei dieser Temperatur werden 200,0 g Wasser eingepumpt und danach auf Raumtemperatur abgekühlt. Die erhaltene Suspension wird abgesaugt und mit 187,3 g Wasser nachgewaschen. Man erhält 251,8 g eines bräunlich-weißen, feinteiligen Feststoffs und 795,7 g einer dunkelbraunen Lösung.

### Beispiel 2

In einem Kolben werden 390,0 g der Lösung aus Beispiel 1 vorgelegt. Man gibt 20,0 g Aktivkohle zu und stellt durch Zudosieren von Salzsäure (37 %ig) einen pH-Wert von 7,0 ein, erhitzt zum Rückfluss, klärt die Reaktionslösung und wäscht mit Wasser nach. Die geklärte Reaktionslösung wird in den Kolben zurückgegeben, auf 0°C abgekühlt und durch Zudosieren von Natronlauge (30 %ig) ein pH von 9,6 eingestellt. Jetzt werden bei 0 bis 5°C Acetanhydrid und Natronlauge simultan so eindosiert, dass der pH-Wert im Bereich von 9,3 bis 9,7 bleibt. Es werden in 1 Stunde 39,8 g Acetanhydrid und 75,9 g Natronlauge (30 %ig) zudosiert. Danach wird 0,5 Stunde bei der gleichen Temperatur nachgerührt. Danach wird bei 5°C durch Zugabe von Salzsäure ein pH-Wert von 1 eingestellt. Dabei fällt das Produkt als weißer Niederschlag aus. Das Produkt wird auf einer Glasfilternutsche abgesaugt und mit Wasser nachgewaschen. Der Niederschlag wird im Vakuumtrockenschrank getrocknet. Man erhält 38,7 g 3-Acetoxy-2-methylbenzoesäure (Gehalt 95,0 %). Das entspricht einer isolierten Ausbeute bezogen auf Trinatrium-1,3,5-naphthalintrisulfonat von 67 % d.Th.

### Beispiel 3

In einem Nickelautoklaven werden in ein Gemisch aus 356,0 g wässriger Natronlauge (45 %ig); 126,0 g Natriumhydroxid-Pastillen und 127,0 g Calciumhydroxid 300,0 g Trinatrium-1,3,5-naphthalintrisulfonat (70,9 %ig, berechnet als freie Säure) so eingerührt, dass eine gut rührbare, breiartige Suspension entsteht. Der Autoklav wird verschlossen und auf 310°C aufgeheizt. Der Innendruck (Eigendruck) steigt dabei auf 50 bar. Es wird 5 Stunden bei der Reaktionstemperatur nachgerührt und danach auf 90°C abgekühlt. Bei dieser Temperatur werden 200,0 g Wasser eingepumpt und danach auf Raumtemperatur abgekühlt. Die erhaltene Suspension wird abgesaugt und mit 609,6 g Wasser nachgewaschen. Man erhält 295,0 g eines bräunlich-weißen, feinteiligen Feststoffs und 1388,8 g einer dunkelbraunen Lösung.

### Beispiel 4

In einem Kolben werden 300,0 g der Lösung aus Beispiel 3 vorgelegt. Man gibt 5 g Aktivkohle zu und stellt durch Zudosieren von Salzsäure (37 %ig) einen pH-Wert von 7,0 ein, erhitzt zum Rückfluss, klärt die Reaktionslösung und wäscht mit Wasser nach. Die geklärte Reaktionslösung wird in den Kolben zurückgegeben, auf 0°C abgekühlt und durch Zudosieren von Natronlauge (30 %ig) ein pH von 9,6 eingestellt. Jetzt werden bei 0 bis 5°C Acetanhydrid und Natronlauge simultan so eindosiert, dass der pH-Wert im Bereich von 9,3 bis 9,7 bleibt. Es werden in 1 Stunde 14,8 g Acetanhydrid und 22,3 g Natronlauge (30 %ig) zudosiert. Danach wird 0,5 Stunde bei der gleichen Temperatur nachgerührt. Danach wird bei 5°C durch Zugabe von Salzsäure ein pH-Wert von 1 eingestellt. Dabei fällt das Produkt als weißer Niederschlag aus. Das Produkt wird auf einer Glasfiltemutsche abgesaugt und mit Wasser nachgewaschen. Der Niederschlag wird im Vakuumtrockenschrank getrocknet. Man erhält 17,4 g 3-Acetoxy-2-methylbenzoesäure (Gehalt 98,2 %). Das entspricht einer isolierten Ausbeute bezogen auf Trinatrium-1,3,5-naphthalintrisulfonat von 71 %d.Th.

### Beispiel 5 (3-Acetoxy-2-methylbenzoesäure)

In einem Planschliffbecher werden 320,0 g Wasser und 120,0 g Natronlauge (w = 30 %) 80,0 g 3-Hydroxy-2-methylbenzoesäure (Gehalt 97,78 %) vermengt. Dabei stellt sich ein pH von 6,9 ein. Die klare Lösung wird auf 0°C abgekühlt und durch Zudosieren von Natronlauge (w = 30 %) auf pH = 9 eingestellt. Jetzt werden bei 0 - 5°C Acetanhydrid und Natronlauge simultan so eindosiert, dass der pH-Wert im Bereich von 8,8 - 9,2 bleibt. Es werden in 2,5 h 112,6 g Acetanhydrid und 172,25 g Natronlauge (w = 30 %) zudosiert. Danach wird 1,5 h nachgerührt.

In einem zweiten Planschliffbecher werden 150,0 g Wasser vorgelegt und auf 0°C abgekühlt. Hierzu dosiert man simultan das Reaktionsgemisch des ersten Bechers und Salzsäure (w = 37 %) bei maximal 5°C so ein, dass ein pH von 3,8 - 4,2 eingehalten wird. Dabei fällt das Produkt als weißer Niederschlag aus. Durch Nachdosieren von Salzsäure (w = 37 %) wird der pH auf eingestellt. Das Produkt wird abgesaugt und mit 500,0 g Wasser nachgewaschen. Der Niederschlag wird im Vakuumtrockenschrank 16 h bei 60°C getrocknet. Man erhält 172,6 g 3-Acetoxy-2-methylbenzoesäure (Gehalt 99,9 %). Das entspricht 99,28 % d.Th.

### Beispiel 6 (3-Acetoxy-2-methylbenzoylchlorid)

In einem Kolben werden unter Stickstoff 100,0 g Thionylchlorid vorgelegt und auf 60°C aufgeheizt. Dazu tropft man eine 170°C heiße Schmelze von 100,0 g 3-Acetoxy-2-methylbenzoesäure (AMBS) in einer Stunde zu. Die Reaktion setzt unter Gasentwicklung (SO₂, HCl) sofort ein. Nach Beendigung der Zugabe wird innerhalb einer Stunde zum Rückfluss (80°C) erhitzt und bei dieser Temperatur bis zum Abklingen der Gasentwicklung (ca. 1 h) gerührt. Die Lösung wird unter Stickstoff auf ca. 50°C abgekühlt und der Rückflusskühler durch eine Vigreux-Kolonne ersetzt. Es werden bei Normaldruck (Sumpf bis 170°C) 31,9 g Thionylchlorid abdestilliert und der zurückbleibende Sumpf im Vakuum (15 mbar) fraktioniert. Man erhält als Destillat 103,8 g 3-Acetoxy-2-methylbenzoylchlorid (98,5 %). Das entspricht 95,5 % d.Th.

### Beispiel 7 (3-Acetoxy-2-methylbenzoylchlorid)

In einem Kolben werden unter Stickstoff in 140,0 g Xylol 60,0 g 3-Acetoxy-2-methylbenzoesäure suspendiert. Die Suspension wird auf 70°C aufgeheizt und 42,9 g Thionylchlorid zugetropft. Man heizt weiter auf. Bei ca. 75°C setzt eine lebhafte Gasentwicklung (SO₂, HCl) ein. Die Sumpftemperatur steigt kontinuierlich an und erreicht nach ca. 4 h 108°C. Die Lösung wird unter Stickstoff auf ca. 50°C abgekühlt und der Rückflusskühler durch eine Vigreux-Kolonne ersetzt. Es werden bei Normaldruck und später im Vakuum erst Xylol und Thionylchloridreste und dann das Produkt überdestilliert. Das Produkt destilliert bei 15 mbar und 147,0 - 148,0°C über. Man erhält 59,6 g 3-Acetoxy-2-methylbenzoylchlorid (97,3 %). Das entspricht 91,0 % d.Th.

## Patentansprüche

1. Verfahren zur Herstellung von Verbindungen der Formel (I), 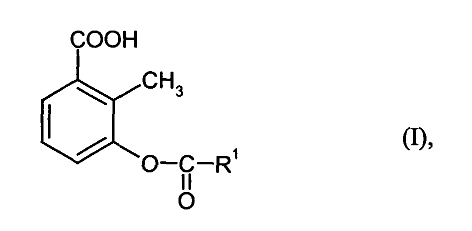 in der
R¹ für C₁-C₂ₒ-Alkyl, C₇-C₂₀-Arylalkyl, C₁₃-C₂₀-Diarylalkyl oder Reste der Formeln (IIa) oder (IIb) steht
A-OR² (IIa)
A-NR³R⁴ (IIb)
in der A jeweils für einen C₁-C₄-Alkylenrest und R² sowie R³ und R⁴ unabhängig voneinander jeweils für Methyl, Ethyl und Isopropyl stehen,
**dadurch gekennzeichnet ist, dass**
a) Verbindungen der Formel (III) 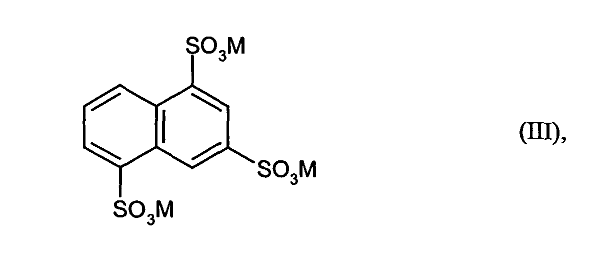 in der M jeweils unabhängig für Wasserstoff, Ammonium, ein Alkalimetall oder ein halbes Äquivalent eines Erdalkalimetalls steht,
mit Alkalimetallhydroxid in Gegenwart von Wasser umgesetzt werden und
b) die in Schritt a) erhaltenen Reaktionsmischungen teilweise neutralisiert werden und
c) die in Schritt b) erhaltenen Reaktionsmischungen mit Verbindungen der Formeln (IVa), oder (IVb)
R¹-COX (IVa)
(R¹-CO)₂O (IVb)
in denen die Reste R¹ jeweils unabhängig voneinander die oben genannte Bedeutung besitzen
umgesetzt werden und
d) die in Schritt c) erhaltenen Reaktionsmischungen angesäuert werden.

2. Verfahren nach Anspruch 1, **dadurch gekennzeichnet, dass** Schritt a) in Gegenwart von Erdalkalimetallhydroxid durchgeführt wird.

3. Verfahren nach Anspruch 1, **dadurch gekennzeichnet, dass** Schritt a) in Gegenwart von Calciumhydroxid durchgeführt wird.

4. Verfahren nach einem oder mehreren der Ansprüche 1 bis 3, **dadurch gekennzeichnet, dass** in Schritt a) 1,3,5-Naphtalintrisulfonsäure oder deren Mono-, Di- oder Tri-Alkalimetallsalze eingesetzt werden.

5. Verfahren nach einem oder mehreren der Ansprüche 1 bis 4, **dadurch gekennzeichnet, dass** in Schritt a) Trinatrium-1,3,5-naphtalin-trisulfonat eingesetzt wird.

6. Verfahren nach einem oder mehreren der Ansprüche 1 bis 5, **dadurch gekennzeichnet, dass** im Schritt b) nach Zugabe von Wasser teilweise neutralisiert wird.

7. Verfahren nach einem oder mehreren der Ansprüche 1 bis 6, **dadurch gekennzeichnet, dass** im Schritt b) durch Filtration, Zentrifugation oder Sedimentation und Dekantieren unlösliche Bestandteile abgetrennt werden.

8. Verfahren nach einem oder mehreren der Ansprüche 1 bis 7, **dadurch gekennzeichnet, dass** im Schritt b) unerwünschten, lösliche Bestandteile durch Extraktion abgetrennt werden.

9. Verfahren nach einem oder mehreren der Ansprüche 1 bis 8, **dadurch gekennzeichnet, dass** im Schritt b) auf einen pH-Wert von 7,5 bis 13 eingestellt wird.

10. Verfahren nach einem oder mehreren der Ansprüche 1 bis 9, **dadurch gekennzeichnet, dass** im Schritt c) Acetanhydrid eingesetzt wird.

11. Verfahren nach einem oder mehreren der Ansprüche 1 bis 10, **dadurch gekennzeichnet, dass** im Schritt c) die molare Verhältnis an Verbindung der Formel (IVa) oder (IVb) zur eingesetzten Verbindung der Formel (II) 0,8:1 bis 10:1 beträgt.

12. Verfahren nach einem oder mehreren der Ansprüche 1 bis 11, **dadurch gekennzeichnet, dass** im Schritt c) der pH-Wert im Laufe der Reaktion zwischen 7,5 und 13 gehalten wird.

13. Verfahren nach einem oder mehreren der Ansprüche 1 bis 12, **dadurch gekennzeichnet, dass** im Schritt d) auf einen pH-Wert von 3,5 oder weniger angesäuert wird.

14. Verfahren nach Anspruch 13, **dadurch gekennzeichnet, dass** im Schritt d) zunächst auf einen pH-Wert von über 3,5 bis unter 8 angesäuert und unerwünschte, lösliche Bestandteile extraktiv entfernt werden und die Reaktionsmischung dann weiter angesäuert wird.

15. Verfahren nach einem oder mehreren der Ansprüche 1 bis 14, **dadurch gekennzeichnet, dass** die Verbindungen der Formel (I) durch Extraktion mit organischem Lösungsmittel, Filtration, Zentrifugation oder Sedimentation und Dekantieren gewonnen werden.

16. Verwendung von Verbindungen der Formel (I) die nach einem oder mehreren der Ansprüche 1 bis 15 hergestellt wurden in einem Verfahren zur Herstellung von Arzneimitteln wie insbesondere HIV-Proteaseinhibitoren und Agrochemikalien wie insbesondere Pflanzenschutzmitteln und Insektiziden oder Zwischenprodukten davon oder zur Herstellung der analogen aktivierten Säurederivate wie insbesondere den Säurechloriden und Säurebromiden.
